# EUROPEAN PATENT SPECIFICATION

(11) **EP 3 203 901 B1**
(45) Date of publication and mention of the grant of the patent: **28.06.2023**
(21) Application number: 15787150.0
(22) Date of filing: 09.10.2015
(51) Int. Cl.: A61B 5/022, A61B 5/024, A61B 5/00

(54) **NON-INVASIVE BLOOD PRESSURE MONITOR, A METHOD OF OPERATING THE SAME, AND A COMPUTER PROGRAM IMPLEMENTING SAID METHOD**
NICHTINVASIVER BLUTDRUCKMONITOR, VERFAHREN ZUM BETRIEB DAVON UND COMPUTERPROGRAMM ZUR DURCHFÜHRUNG DIESES VERFAHRENS
MONITEUR DE PRESSION SANGUINE NON INVASIVE, SON PROCÉDÉ DE FONCTIONNEMENT ET PROGRAMME D'ORDINATEUR POUR RÉALISER LEDIT PROCÉDÉ

(30) Priority: 10.10.2014 EP 14188411
(43) Date of publication of application: 16.08.2017
(73) Proprietor: Koninklijke Philips N.V., 5656 AG Eindhoven (NL)
(72) Inventor: MÜHLSTEFF, Jens, NL-5656 AE Eindhoven (NL); VAN DEN HEUVEL, Teun, NL-5656 AE Eindhoven (NL); BRESCH, Erik, NL-5656 AE Eindhoven (NL); SCHMITT, Lars, NL-5656 AE Eindhoven (NL); WOEHRLE, Dieter, NL-5656 AE Eindhoven (NL)
(74) Representative: Philips Intellectual Property & Standards
(86) International application number: PCT/EP2015/073407
(87) International publication number: WO 2016/055614

(56) References cited:
- EP-A1- 2 759 257
- WO-A2-2009/125349
- JP-A- 2001 333 889
- US-A1- 2012 157 791
- US-A1- 2012 330 112

## Description

### TECHNICAL FIELD OF THE INVENTION

The invention relates to a non-invasive blood pressure (NIBP) monitor and a method of operating the same, and in particular relates to improving the comfort of the blood pressure measurements obtained by such a monitor.

### BACKGROUND TO THE INVENTION

Arterial blood pressure (BP) is one of the most important vital signs and is widely used in clinical practice. Non-invasive arterial blood pressure (NIBP) is usually measured by slowly varying the pressure in a cuff that is wrapped around the upper arm of a subject. The BP is determined either by measuring sound distal from the cuff (the auscultatory method, based on Korotkoff sounds) or by measuring pressure pulsations in the cuff caused by volume pulsations of the arm and brachial artery and extracting features from the envelope of these pressure pulses (the oscillometric method). The oscillometric method is easily automated and is widely used.

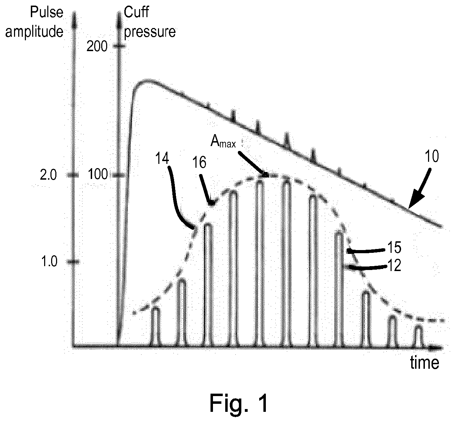

The principle behind a typical oscillometric method is illustrated by Figure 1, which shows a graph of cuff pressure 10, and a processed high pass filtered trace 12 of this cuff pressure, versus time. The left-hand y-axis shows pulse amplitude, the right-hand y-axis shows cuff pressure, and the x-axis shows time. To perform a NIBP measurement using the oscillometric method, first the cuff pressure 10 is ramped up until it is sufficiently larger than systolic blood pressure. After ramp up, the cuff is deflated (in Figure 1 the deflation is done gradually, but step wise deflation is also possible). During the deflation, small oscillations in cuff pressure occur, caused by volume changes in the bladder of the cuff, which are in turn caused by volume changes in the brachial artery. The measured cuff pressure 10 is high pass filtered, and the resulting trace 12 shows the cuff pressure oscillations due to volume changes in the brachial artery. An envelope 14 of the oscillation amplitudes is determined. The maximum Aₘₐₓ of this pulse envelope 14 is taken as a reference point for determining the systolic 16 and diastolic pressure 15. The systolic pressure 16 is determined as the cuff pressure where the pressure oscillation is approximately 0.8 times the maximum amplitude Aₘₐₓ at a pressure higher than the pressure at the reference point. The diastolic pressure 15 is determined as the cuff pressure where the pressure oscillation is approximately 0.55 times the maximum amplitude Aₘₐₓ at a pressure lower than the pressure at the reference point. These ratios are based on empirical values (see, e.g., LA Geddes et. al., Annals of Biomedical Engineering 10 pp 271-280, 1982). The exact algorithms that are employed by manufacturers of blood pressure devices to determine systolic and diastolic pressures are usually trade secrets.

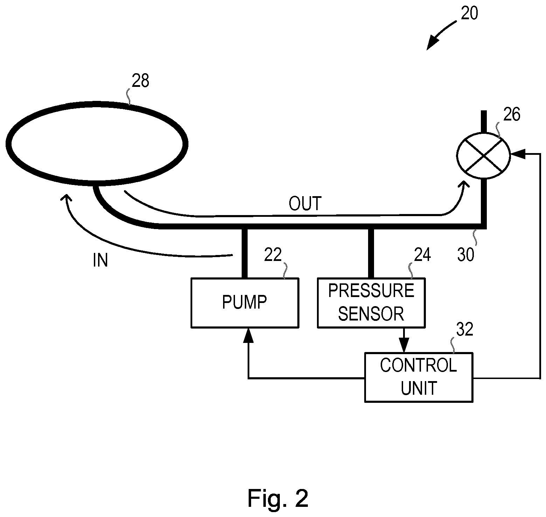

The typical monitor 20 used for acquiring oscillometric NIBP measurements is illustrated in Figure 2. A pump 22, a pressure sensor 24, and a valve 26 are connected to a cuff 28 by tubing 30. A control unit 32 is connected to the pump 22 and the valve 26 to control the operation of those components, and is also connected to the pressure sensor 24 in order to receive the signal representing the pressure of the gas in the cuff 28 (the 'pressure signal'). The control unit 32 runs the algorithm that controls the pump 22 and valve 26 and processes the pressure signal from the pressure sensor 24 to determine the BP measurement. During execution of the oscillometric method the pump 22 blows air into the cuff 28, thereby inflating it. The pressure sensor 24 measures the gas pressure in the system (and therefore the pressure of the gas in the cuff 28) and outputs a signal representing the pressure in the cuff 28 (referred to as the 'pressure signal'). When a pressure larger than systolic pressure is reached, the pump 22 is disabled or switched off, the valve 26 is opened and slow (or step wise) deflation occurs, during which the cuff pressure is continuously measured and the measurements (pressure signal) stored. The pump 22 and valve 26 are controlled by control unit 32, which also receives the cuff pressure measurements and calculates the pulse envelope and the systolic and diastolic pressure using these measurements. In practice the monitor 20 may comprise multiple sensors and valves for safety reasons.

The operation of the typical monitor 20 is often uncomfortable for the subject (and in some cases is painful), since the arm is compressed with an external pressure. In a clinical or hospital (or even home) setting where blood pressure measurements need to be obtained through the day and night, the taking of a blood pressure measurement by the monitor 20 will often disturb the sleep of the subject. NIBP monitors originally developed for high acuity subjects (e.g. those in an intensive care unit (ICU)) were optimized for accuracy and precision, but not the comfort of the subject.

In a home setting, it has been found that NIBP measurements have a relatively low acceptance by subjects (e.g. the subjects do not comply with the required measurement schedule or do not perform the measurements properly), which in some cases is due to the pain caused by the inflation of the cuff (which can relate to the duration that the cuff is inflated for and/or the peak pressure in the cuff), irritation of skin under the cuff (particularly on NIBP monitors that are continuously worn by a subject), haematomas, and disturbance of the sleep of the subject.

The comfort of the NIBP measurement can be improved in any or all of three areas: the total measurement time (where a reduction is desired), the maximum cuff pressure reached (where a lower maximum pressure is desired) and the integral of cuff pressure over time (where a smaller integral is desired). Of course, this increase in comfort should not come at the expense of the accuracy of the NIBP measurement beyond acceptable limits.

In addition to the types of monitor described above in which the BP is measured using envelope detection during deflation of the cuff (which can typically take around 45 seconds), monitors have been developed that can measure the BP while the cuff is being inflated. This can reduce the total measurement time (in some cases to around 20 seconds), since the deflation stage can be very quick once the BP measurement has been obtained, and therefore can result in a measurement that is more comfortable for the subject. However, there is further scope for improving the comfort of these inflation-based BP measurements.

Document JP 2001 333889 A relates to a sphygmomanometer that can set an appropriate pressing amount in spite of any conditions including pressing speed. Document US 2012/157791 A1 describes an adaptive time-domain filtering for improved blood pressure estimation. US 2012/330112 A1 discloses a patient monitoring system. WO 2009/125349 A2 relates to a multi-sensor apparatus and method for monitoring circulatory parameters. EP 2 759 257 A1 describes a method, logic unit and system for determining a parameter representative for the patient's volume responsiveness.

Therefore there is a need for an NIBP monitor and method of operating the same that measures the blood pressure during inflation of the cuff and that is more comfortable for the subject than conventional monitors.

### SUMMARY OF THE INVENTION

The invention is defined by a method according to claim **1,** a computer program product according to claim **7** and a NIBP monitor according to claim **8**. Further embodiments are defined by dependent claims **2-6, 9-12.**

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a graph of cuff pressure versus time measured using a conventional oscillometric NIBP monitor;
Figure 2 shows a block diagram of a conventional oscillatory NIBP monitor;
Figure 3 is a flow chart illustrating an exemplary method for performing an inflation-based BP measurement;
Figure 4 is a block diagram of an NIBP monitor according to an embodiment of the invention;
Figure 5 is a flow chart illustrating a method of operating a NIBP monitor to obtain an inflation-based BP measurement according to an aspect of the invention; and
Figure 6 is a graph illustrating the improvements in BP measurements provided by the invention.

### DETAILED DESCRIPTION OF THE PREFERRED EMBODIMENTS

As described above, measuring the blood pressure (BP) of a subject during inflation of a cuff, rather than during deflation of the cuff from a peak pressure that is sufficient to prevent blood flow in the limb, allows the BP measurement to be completed more quickly, which helps to improve the comfort of the BP measurement for the subject.

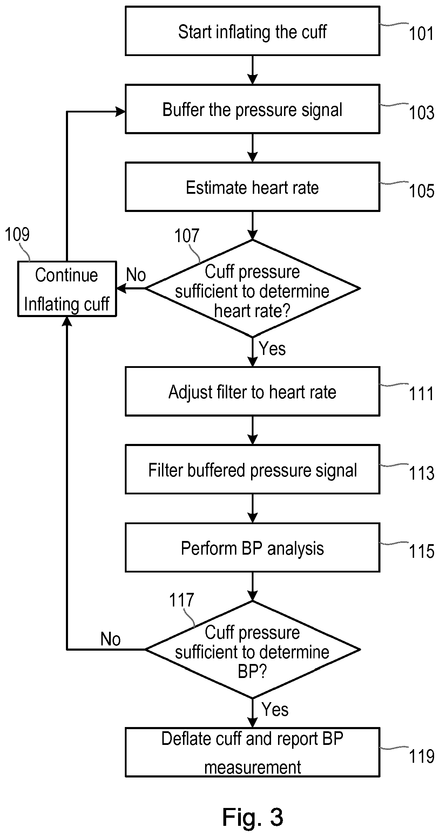

The flow chart in Figure 3 illustrates an exemplary method for performing an inflation-based measurement using the monitor 20 shown in Figure 2. Briefly, in this method, information about the subject's heart rate is obtained from the pressure signal and used to adapt a filter that is applied to the pressure signal in order to obtain the BP measurement.

In step 101 inflation of the cuff 28 is started, and the pressure signal representing the pressure in the system is buffered (step 103). In order to obtain a reliable BP measurement, the subject's blood pressure range (diastolic to systolic) needs to be sampled over a certain number of heart cycles (beats). This certain number of heart cycles puts an upper limit on the rate at which the cuff can be inflated from the diastolic pressure to the systolic pressure (since if the cuff is inflated too quickly there will not be enough heart cycles in the signal representing the pressures between diastolic and systolic pressure to obtain a reliable BP measurement). Thus, in step 101 the cuff 28 is inflated at a rate that will allow a sufficient number of heart cycles in the diastolic to systolic range to be measured (typically this rate is set based on an assumption of the lowest possible heart rate in order to ensure that a sufficient number of heart cycles are captured).

In step 105, the buffered pressure signal is processed to determine the heart rate of the subject. This step typically requires the cuff to be at a certain pressure for the heart rate signal to be observed, and a substantial length or duration of pressure signal (e.g. covering a few heart beats) is required in order to be able to extract the heart rate information. If the pressure in the cuff is not yet sufficient to determine the heart rate (as determined in step 107), then no heart rate will be determined in step 105, and the method passes to step 109 in which inflation of the cuff 28 continues and the pressure signal continues to be buffered (step 103). In some embodiments, step 105 can comprise estimating the autocorrelation function of the buffered pressure signal and then finding a peak in that function that most likely corresponds to the subject's heart rate. In some cases the amplitude of the peak can be compared to a threshold to determine if the peak has a sufficient magnitude to represent the heart rate. If the amplitude of the peak exceeds the threshold, the peak can be used as the estimate of the heart rate.

If the heart rate can be extracted from the pressure signal in step 105, the method passes to step 111 in which the heart rate is used to adapt a filter that is to be applied to the pressure signal. Once the filter has been adapted to the heart rate of the subject, the buffered pressure signal can be filtered (step 113) and an analysis of the filtered pressure signal performed to determine the BP (step 115). The filter is a high-pass filter, similar to the high-pass filter used in the traditional oscillometric method. The heart rate (HR) is used to adapt the frequency characteristics of the filter, e.g. the cut-off frequency. In some embodiments, the cut-off frequency can be set to the heart rate (e.g. 60 beats per minute, bpm = 1 Hz), but in other embodiments the cut-off frequency may be (slightly) higher or lower than the heart rate (depending on details of the filter design and other processing steps). Generally, the higher the heart rate, the higher the cut-off frequency of the filter (and vice versa). According to the invention, a basic moving average filter is used, whereby the width of the averaging window is set to correspond to the heart period (=1/HR). Running this filter over the pressure signal returns a low-pass filtered pressure signal, which is then subtracted from the original pressure signal to obtain the high-pass filtered pressure signal.

If the pressure in the cuff 28 is not yet sufficient to determine the BP (e.g. if the cuff pressure has not yet reached the systolic pressure) then the inflation of the cuff continues (step 109). At this stage, as the heart rate has already been determined, it may not be necessary to continue processing the pressure signal to extract the current heart rate (step 105) and adapt the filter (step 111), so the method can return to step 113 after step 109 and the filter can be applied to newly buffered pressure signal data. In other implementations, the extraction of the heart rate and adaptation of the filter can be a continuous process, in which case the method can return to step 103. If at step 115 a BP measurement is determined from the pressure signal, then the method passes to step 119 in which the inflation of the cuff 28 by the pump 22 is stopped, the cuff 28 is deflated and the BP measurement can be reported to the subject, other operator of the monitor 20 or a remote computer or base unit that collates and stores the BP measurement information for the subject.

Thus, in this method, obtaining a BP measurement during inflation of the cuff 28 requires information about the subject's heart rate in order to be able to filter the pressure signal appropriately. Since estimating the heart rate from the pressure signal during inflation requires a certain pressure in the cuff for a substantial length of time (e.g. covering a few heart beats), there can be a delay in being able to perform the BP measurement processing while the heart rate information is extracted (and hence a delay in being able to determine that inflation of the cuff 28 can be stopped), by which time the cuff 28 may have been inflated beyond the maximum pressure required to complete the BP measurement. This can cause discomfort for the subject due to excess pressure and duration of the BP measurement.

Therefore, the invention provides that heart rate information (which is according to the invention the pulse rate of the subject) used to adapt the filter applied to the pressure signal is obtained from a different, separate sensor, rather than being derived from the pressure signal itself. This means that heart rate information is available continuously throughout the inflation of the cuff, and the disadvantages with the method in Figure 3 can be overcome. In particular, the processing of the pressure signal to obtain the BP measurement can start as soon as the inflation of the cuff starts (instead of when a sufficient pressure has been reached in the cuff to enable the heart rate information to be derived), which increases the chances of the inflation of the cuff being stopped at the earliest opportunity (i.e. when the systolic pressure is reached).

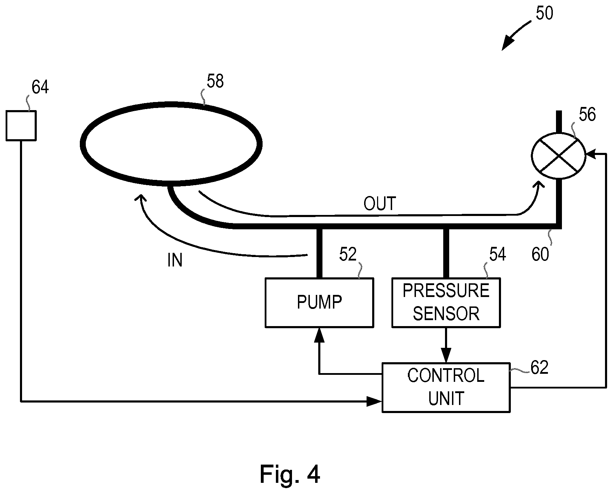

A non-invasive blood pressure (NIBP) monitor according to an embodiment of the invention is shown in Figure 4. The monitor 50 comprises a pump 52, a pressure sensor 54, and a valve 56 that are connected to a cuff 58 by tubing 60. A control unit 62 is connected to the pump 52 and the valve 56 to control the operation of those components, and is also connected to the pressure sensor 54 in order to receive the signal representing the pressure of the gas in the cuff 58 (the 'pressure signal'). The control unit 62 runs the algorithm that controls the pump 52 and valve 56 and processes the pressure signal from the pressure sensor 54 to determine the BP measurement. The control unit 62 can comprise one or more processors that are configured or programmed to control the operation of the components of the NIBP monitor 50 and obtain the BP measurement.

As in the conventional monitor, the pump 52 is for blowing air or other gas into the cuff 58 in order to inflate the cuff 58 and prevent blood flow in the limb around which the cuff 58 is placed. The valve 56 is used to allow air or gas out of the system and thus deflate the cuff 58.

In accordance with an embodiment of the invention, the monitor 50 further comprises a pulse rate sensor 64 that is for measuring the pulse rate of the subject while the cuff 58 is being inflated. The pulse sensor 64 can be any suitable type of sensor that measures the pulse rate of the subject, and that is able to obtain a pulse rate measurement at any required time (e.g. as required by the BP measurement algorithm).

In some embodiments, the sensor 64 is a photoplethysmography (PPG) sensor, an accelerometer or an ECG sensor, although those skilled in the art will be aware of other types of heart rate sensor that can be used (such as a camera, radar, impedance cardiogram, heart sound sensor, etc.). In the case of a PPG sensor, accelerometer and/or ECG sensor, the sensor 64 can comprise the appropriate sensing apparatus, e.g. light source and detector for a PPG sensor, accelerometer, and two or more electrodes for an ECG sensor, and the processing of the signals from those sensors to determine the pulse rate can be performed by the control unit 62. In the case of an accelerometer, the acceleration signal can be processed to extract the movements caused by the beating of the heart/pulses of blood in the circulatory system.

In use, the pulse rate sensor 64 is attached to or otherwise in contact with the appropriate part of the body of the subject in order to measure the pulse rate. It will be appreciated that in some embodiments, the sensor 64 can be integrated with the cuff 58 so the subject only has to place the cuff around their arm in order to start using the monitor 50, whereas in other embodiments the sensor 64 can be separate from the cuff 58 and placed separately on the body of the subject. In some embodiments, there can be a wired connection between the sensor 64 and the control unit 62, whereas in other embodiments, the sensor 64 can communicate with the control unit 62 wirelessly.

It will be appreciated that Figure 4 only shows the components required to illustrate this aspect of the invention, and in a practical implementation the NIBP monitor 50 will comprise additional components to those shown. For example, the monitor 50 may comprise multiple pressure sensors 54 and valves 56 for safety reasons, a battery or other power supply for powering the monitor 50, a memory module for storing program code and/or the BP measurements, a communication module for enabling the BP measurements to be communicated to a base unit for the monitor 50 or a remote computer, and/or one or more user interface components that allow a user (e.g. the subject or healthcare professional) to interact and control the monitor). Also, in embodiments of the invention the pulse rate sensor 64 need not form part of the monitor 50 as such, with instead the monitor 50 being arranged to obtain information on the pulse rate of the subject from a separately provided pulse rate sensor 64.

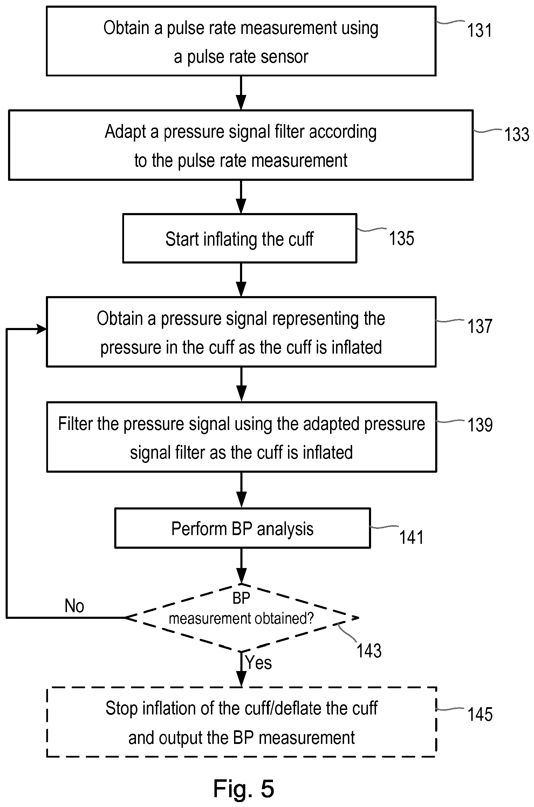

The flow chart in Figure 5 illustrates a method of obtaining a measurement of the blood pressure of a subject during inflation of a cuff according to the invention. This method can be implemented by a NIBP monitor 50 as shown in Figure 4, and it will be appreciated that in some embodiments the NIBP monitor 50 can comprise computer program code for enabling the control unit 62 to perform the method.

In the first step, step 131, the pulse rate sensor 64 is used to obtain a measurement of the pulse rate of the subject. This step is first performed at the start of the BP measurement process, preferably just prior to starting the inflation of the cuff 58 by pump 52, although in some embodiments it can be performed as, or just after, inflation of the cuff 58 is started. In any case, the measurement of the pulse rate is available much earlier in the process than in the exemplary method above in which the pulse rate information has to be derived from the pressure signal.

Once the measurement of the pulse rate has been obtained, the filter that is applied to the pressure signal as part of the process for determining the BP measurement is adapted according to the pulse rate measurement (step 133). As in the method of Figure 3, in step 133 the filter can be a high-pass filter, similar to the high-pass filter used in the traditional oscillometric method. The pulse rate (PR) is used to adapt the frequency characteristics of the filter, e.g. the cut-off frequency. In some embodiments, the cut-off frequency can be set to the heart rate (e.g. 60 beats per minute, bpm = 1 Hz), but in other embodiments the cut-off frequency may be set (slightly) higher or lower than the heart rate (depending on details of the filter design and other processing steps). Generally, the higher the heart rate, the higher the cut-off frequency of the filter (and vice versa). According to the invention, a basic moving average filter is used, whereby the width of the averaging window is set to correspond to the heart period (=1/PR). Running this filter over the pressure signal returns a low-pass filtered pressure signal, which is then subtracted from the original pressure signal to obtain the high-pass filtered pressure signal.

The inflation of the cuff 58 is started (step 135) and a pressure signal representing the pressure in the cuff 58 is obtained (step 137).

Next, this pressure signal is filtered using the adapted filter (step 139). This filtering is performed during inflation of the cuff 58, and preferably in real time as the pressure signal is obtained (or as near to real time as is possible). The filtered pressure signal is then analyzed to determine the BP measurement (step 141). Those skilled in the art will be aware of various techniques for performing this analysis, and therefore further details are not provided herein. Again, this step is performed during inflation of the cuff 58, and preferably in real time (or as near to real time as is possible).

If a BP measurement cannot be obtained from the pressure signal (e.g. if the pressure in the cuff 58 has not yet reached the systolic blood pressure in the subject), then following step 143 the inflation of the cuff 58 continues and the method returns to step 137 where the pressure signal continues to be obtained, filtered and analyzed. If a BP measurement is obtained in step 141, the method passes to step 145 in which the inflation of the cuff 58 is stopped and the deflation of the cuff 58 is started (e.g. by opening valve 56). Since the BP measurement has been obtained, the deflation is preferably performed as fast as possible. The deflation is preferably initiated as soon as or immediately after the BP measurement has been obtained. The BP measurement can be reported to the subject, other operator of the monitor 50 or a remote computer or base unit that collates and stores the BP measurement information for the subject.

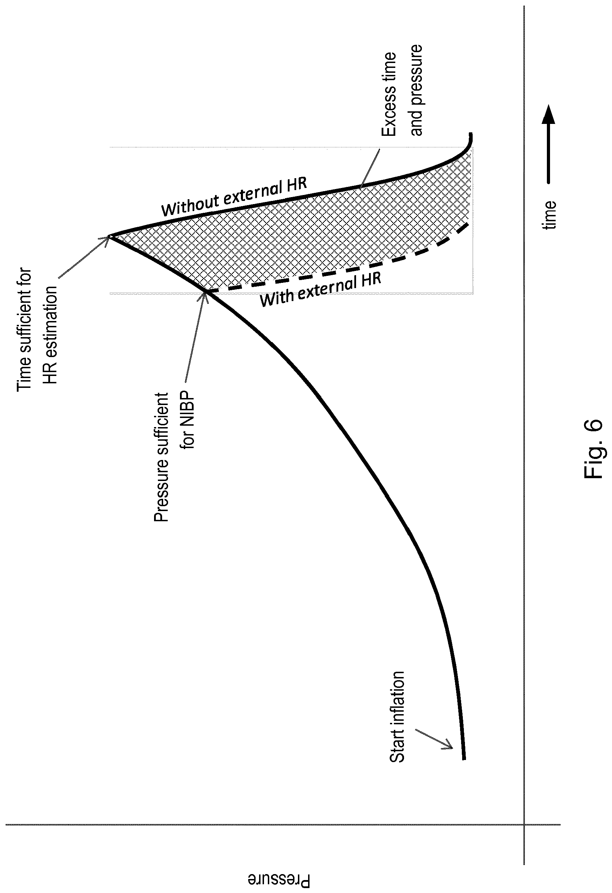

The graph in Figure 6 illustrates the reduction in measurement time and maximum pressure that is possible with an external heart rate (HR)/pulse rate measurement according to the invention shown in Figure 5. It can be seen that if the heart rate information is obtained from the pressure signal itself, by the time the heart rate information has been derived, the pressure in the cuff may have already exceeded that required to complete the BP measurement, which leads to a longer measurement time and higher peak pressure in the cuff than is required.

Instead, by obtaining the heart rate information (particularly the pulse rate of the subject) from a different, separate sensor 64 (and therefore not from the pressure signal), the heart rate information is available continuously throughout the inflation of the cuff 58, which means that the processing of the pressure signal to obtain the BP measurement can start as soon as the inflation of the cuff 58 starts (and thus always starts before the systolic pressure is reached in the cuff 58) and therefore the BP measurement can be obtained at the earliest opportunity.

In some embodiments, since the pulse rate of the subject can change over the course of the BP measurement process, the pulse rate sensor 64 can also be used to obtain measurements of the pulse rate of the subject during inflation of the cuff 58, and those measurements can be used to adapt the filter to the current pulse rate of the subject. This helps to ensure that the filter is up to date for the current pulse rate of the subject and therefore provide a BP measurement that is as accurate as possible.

In some embodiments, if for any reason the analysis in step 141 is unable to obtain a BP measurement (or a BP measurement that is reliable), for example if the subject moves during the measurement process or experiences an arrhythmia, the control unit 62 can control the NIBP monitor 50 to perform a conventional deflation-based BP measurement. Thus, when the systolic pressure has been exceeded (or when a preset maximum inflation pressure has been reached without being able to obtain a BP measurement) the control unit 62 can control the pump 52 to stop inflating the cuff 58, and operate the valve 56 to release pressure from the cuff 58 in a controlled manner. The pressure signal from the pressure sensor 54 obtained while the cuff 58 deflates is analyzed by the control unit 62 using a conventional deflation-based algorithm to determine the blood pressure of the subject.

There is therefore provided an NIBP monitor and a method of operating the same that measures the blood pressure of a subject during inflation of the cuff and that is more comfortable for the subject than measurements made according to conventional methods or by conventional monitors.

In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of obtaining a measurement of the blood pressure of a subject during inflation of a cuff using a non-invasive blood pressure, NIBP, monitor comprising a cuff that is to be placed around a limb of the subject, a pump for inflating the cuff, and a pressure sensor for measuring the pressure in the cuff, and further including use of a pulse rate sensor, wherein the pulse rate sensor is a different sensor to the pressure sensor, the method comprising:
obtaining (131) information on the pulse rate of the subject from the pulse rate sensor;
adapting (133) a pressure signal filter according to the obtained information on the pulse rate of the subject, wherein the pressure signal filter is a moving average filter with an averaging window width set to correspond to the heart period obtained from the information on the pulse rate of the subject;
starting inflation of the cuff;
obtaining (137) a pressure signal from the pressure sensor, the pressure signal representing the pressure in the cuff as the cuff is inflated;
filtering (139) the pressure signal using the adapted pressure signal filter as the pressure signal is obtained, the filtering comprising the steps of:
running the moving average filter over the pressure signal, and
subtracting the resulting pressure signal from the original pressure signal to obtain the filtered pressure signal; and
processing (141), as the filtered pressure signal is obtained, the filtered pressure signal to obtain a blood pressure measurement for the subject.

2. A method as claimed in claim 1, wherein step of obtaining (131) information on the pulse rate of the subject from the pulse rate sensor is performed prior to starting the inflation of the cuff, and wherein preferably the step of adapting (133) the pressure signal filter is also performed prior to starting the inflation of the cuff.

3. A method as claimed in claim 2, wherein the steps of obtaining (131) information on the pulse rate of the subject from the pulse rate sensor and adapting (133) the pressure signal filter are also performed during inflation of the cuff.

4. A method as claimed in any of claims 1-3, the method further comprising the step of deflating (145) the cuff once the blood pressure measurement has been obtained.

5. A method as claimed in any of claims 1-4, the method further comprising the step of performing (145) a measurement of the blood pressure during deflation of the cuff if the step of processing (141) the filtered pressure signal to obtain a blood pressure measurement for the subject does not provide a measurement of the blood pressure or an acceptable measurement.

6. A method as claimed in any of claims 1-5, wherein the step of adapting (133) the pressure signal filter comprises adapting the frequency characteristics of the filter according to the obtained information on the pulse rate of the subject.

7. A computer program product comprising a computer readable medium having computer readable code embodied therein, the computer readable code being configured such that, on execution by a suitable computer, processor or control unit that is for use with a non-invasive blood pressure, NIBP, monitor comprising a cuff that is to be placed around a limb of the subject, a pump for inflating the cuff and a pressure sensor for measuring the pressure in the cuff, wherein the NIBP monitor is used with a pulse rate sensor, wherein the pulse rate sensor is a different sensor to the pressure sensor, the computer, processor or control unit is caused to perform the method of any of claims 1-6.

8. A non-invasive blood pressure, NIBP, monitor (50) for measuring the blood pressure of a subject during inflation of a cuff (58) when the NIBP monitor (50) is used with a pulse rate sensor (64), the NIBP monitor comprising a cuff (58) that is to be placed around a limb of the subject, a pump (52) for inflating the cuff (58) and a pressure sensor (54) for measuring the pressure in the cuff (58), wherein the pulse rate sensor (64) is a different sensor to the pressure sensor (54), the NIBP monitor (50) further comprising:
a control unit (62) that is configured to:
obtain information on the pulse rate of the subject from the pulse rate sensor (64);
adapt a pressure signal filter according to the obtained information on the pulse rate of the subject, wherein the pressure signal filter is a moving average filter with an averaging window width set to correspond to the heart period obtained from the information on the pulse rate of the subject;
control the pump (52) to start inflating the cuff (58);
obtain a pressure signal from the pressure sensor (54), the pressure signal representing the pressure in the cuff (58) as the cuff (58) is inflated;
filter the pressure signal using the adapted pressure signal filter as the pressure signal is obtained, the filtering comprising the steps of:
running the moving average filter over the pressure signal, and
subtracting the resulting pressure signal from the original pressure signal to obtain the filtered pressure signal; and
process the filtered pressure signal as the filtered pressure signal is obtained to obtain a blood pressure measurement for the subject.

9. A NIBP monitor (50) as claimed in claim 8, wherein the pulse rate sensor (64) for measuring the pulse rate of the subject and for providing information on the pulse rate to the control unit (62) is comprised in the NIBP monitor (50).

10. A NIBP monitor (50) as claimed in any of claims 8-9, wherein the control unit (62) is configured to obtain the information on the pulse rate of the subject and, preferably, also to adapt the pressure signal filter, prior to controlling the pump (52) to start inflating the cuff (58).

11. A NIBP monitor (50) as claimed in any of claims 8-10, wherein the control unit (62) is further configured to obtain information on the pulse rate of the subject from the pulse rate sensor (64) and to adapt the pressure signal filter during inflation of the cuff (58).

12. A NIBP monitor (50) as claimed in any of claims 8-11, wherein the control unit (62) is configured to adapt the pressure signal filter by adapting the frequency characteristics of the filter according to the obtained information on the pulse rate of the subject.

## Patentansprüche

1. Verfahren zum Erlangen einer Messung des Blutdrucks eines Subjekts während eines Aufblasens einer Manschette unter Verwendung eines nicht-invasiven Blutdruckmessgeräts (NIBP-Messgerät), umfassend eine Manschette, die um ein Körperglied des Subjekts platziert werden soll, eine Pumpe zum Aufblasen der Manschette und einen Drucksensor zum Messen des Drucks in der Manschette umfasst, und das ferner eine Verwendung eines Pulszahlsensors beinhaltet, wobei der Pulszahlsensor ein anderer Sensor als der Drucksensor ist, das Verfahren umfassend:
Erlangen (131) von Informationen über die Pulszahl des Subjekts von dem Pulszahlsensor; Anpassen (133) eines Drucksignalfilters gemäß den erlangten Information über die Pulszahl des Subjekts, wobei der Drucksignalfilter ein Filter mit gleitendem Mittelwert ist, dessen Mittelungsfensterbreite eingestellt ist, um der Herzperiode zu entsprechen, die aus den Informationen über die Pulszahl des Subjekts erlangt werden; Beginn eines Aufblasens der Manschette; Erlangen (137) eines Drucksignals von dem Drucksensor, wobei das Drucksignal den Druck in der Manschette darstellt, während die Manschette aufgeblasen wird; Filtern (139) des Drucksignals unter Verwendung des angepassten Drucksignalfilters, wenn das Drucksignal erlangt wird,
das Filtern umfassend die folgenden Schritte:
Ausführen des Filters mit gleitendem Mittelwert über das Drucksignal und Subtrahieren des resultierenden Drucksignals von dem ursprünglichen Drucksignal, um das gefilterte Drucksignal zu erlangen; und Verarbeiten (141) des gefilterten Drucksignals, während das gefilterte Drucksignal erlangt wird, um eine Blutdruckmessung für das Subjekt zu erlangen.

2. Verfahren nach Anspruch 1, wobei der Schritt eines Erlangens (131) von Informationen über die Pulszahl des Subjekts von dem Pulszahlsensor vor Beginnen des Aufblasens der Manschette ausgeführt wird, und wobei vorzugsweise der Schritt eines Anpassens (133) des Drucksignalfilters auch vor Beginnen des Aufblasens der Manschette ausgeführt wird.

3. Verfahren nach Anspruch 2, wobei die Schritte eines Erlangens (131) von Informationen über die Pulszahl des Subjekts von dem Pulszahlsensor und eines Anpassens (133) des Drucksignalfilters auch während eines Aufblasens der Manschette ausgeführt werden.

4. Verfahren nach einem der Ansprüche 1-3, Verfahren ferner umfassend den Schritt eines Leerens (145) der Manschette, sobald die Blutdruckmessung erlangt wurde.

5. Verfahren nach einem der Ansprüche 1-4, das Verfahren ferner umfassend den Schritt eines Ausführens (145) einer Messung des Blutdrucks während eines Leerens der Manschette, wenn der Schritt eines Verarbeitens (141) des gefilterten Drucksignals, um eine Blutdruckmessung für das Subjekt zu erlangen, keine Messung des Blutdrucks oder keine annehmbare Messung bereitstellt.

6. Verfahren nach einem der Ansprüche 1-5, wobei der Schritt eines Anpassens (133) des Drucksignalfilters ein Anpassen der Frequenzcharakteristiken des Filters gemäß den erlangten Informationen über die Pulszahl des Subjekts umfasst.

7. Computerprogrammprodukt, umfassend ein computerlesbares Medium, das darin verkörperten computerlesbaren Code aufweist, wobei der computerlesbare Code konfiguriert ist, sodass bei Ausführung durch einen geeigneten Computer, Prozessor oder Steuereinheit, der/die zur Verwendung mit einem nicht-invasiven Blutdruckmessgerät, NIBP-Messgerät, ist, umfassend eine Manschette, die um ein Körperglied des Subjekts platziert werden soll, eine Pumpe zum Aufblasen der Manschette und einen Drucksensor zum Messen des Drucks in der Manschette, wobei das NIBP-Messgerät mit einem Pulszahlsensor verwendet wird, wobei der Pulszahlsensor ein anderer Sensor ist als der Drucksensor, wobei der Computer, der Prozessor oder die Steuereinheit veranlasst wird, das Verfahren nach einem der Ansprüche 1-6 auszuführen.

8. Nicht-invasives Blutdrucküberwachungsgerät, NIBP-Messgerät (50) zum Messen des Blutdrucks einer Subjekt während eines Aufblasens einer Manschette (58), wenn das NIBP-Messgerät (50) mit einem Pulszahlsensor (64) verwendet wird, das NIBP-Messgerät umfassend eine Manschette (58), die um ein Körperglied des Subjekts platziert werden soll, eine Pumpe (52) zum Aufblasen der Manschette (58) und einen Drucksensor (54) zum Messen des Drucks in der Manschette (58), wobei der Pulszahlsensor (64) ein anderer Sensor ist als der Drucksensor (54), das NIBP-Messgerät (50) ferner umfassend:
eine Steuereinheit (62), die zu Folgendem konfiguriert ist:
Erlangen von Informationen über die Pulszahl des Subjekts vom Pulszahlsensor (64); Anpassen eines Drucksignalfilters gemäß den erlangten Information über die Pulszahl des Subjekts, wobei der Drucksignalfilter ein Filter mit gleitendem Mittelwert ist, dessen Mittelungsfensterbreite eingestellt ist, um der Herzperiode zu entsprechen, die aus den Informationen über die Pulszahl des Subjekts erlangt werden; Steuern der Pumpe (52), um ein Aufblasen der Manschette (58) zu beginnen; Erlangen eines Drucksignals von dem Drucksensor (54), wobei das Drucksignal den Druck in der Manschette (58) darstellt, während die Manschette (58) aufgeblasen wird; Filtern des Drucksignals unter Verwendung des angepassten Drucksignalfilters, wenn das Drucksignal erlangt wird, das Filtern umfassend die folgenden Schritte:
Ausführen des Filters mit gleitendem Mittelwert über das Drucksignal, und Subtrahieren des resultierenden Drucksignals von dem ursprünglichen Drucksignal, um das gefilterte Drucksignal zu erlangen; und Verarbeiten des gefilterten Drucksignals, während das gefilterte Drucksignal erlangt wird, um eine Blutdruckmessung für das Subjekt zu erlangen.

9. NIBP-Messgerät (50) nach Anspruch 8, wobei der Pulszahlsensor (64) zum Messen der Pulszahl des Subjekts und zum Bereitstellen von Informationen über die Pulszahl an die Steuereinheit (62) in dem NIBP-Messgerät (50) enthalten ist.

10. NIBP-Messgerät (50) nach einem der Ansprüche 8-9, wobei die Steuereinheit (62) konfiguriert ist, um die Informationen über die Pulszahl des Subjekts zu erlangen und vorzugsweise auch, um den Drucksignalfilter anzupassen, vor einem Steuern der Pumpe (52), um eine Aufblasen der Manschette (58) zu beginnen.

11. NIBP-Messgerät (50) nach einem der Ansprüche 8-10, wobei die Steuereinheit (62) ferner konfiguriert ist, um Informationen über die Pulszahl des Subjekts von dem Pulszahlsensor (64) zu erlangen und den Drucksignalfilter während eines Aufblasens der Manschette (58) anzupassen.

12. NIBP-Messgerät (50) nach einem der Ansprüche 8 bis 11, wobei die Steuereinheit (62) konfiguriert ist, um den Drucksignalfilter anzupassen, indem sie die Frequenzcharakteristik des Filters gemäß den erlangten Informationen über die Pulszahl des Subjekts anpasst.

## Revendications

1. Procédé d'obtention d'une mesure de la pression sanguine d'un sujet pendant le gonflage d'un brassard en utilisant un moniteur de pression sanguine non invasif, NIBP, comprenant un brassard qui doit être placé autour d'un membre du sujet, une pompe pour gonfler le brassard et un capteur de pression pour mesurer la pression dans le brassard, et incluant en outre l'utilisation d'un capteur de fréquence du pouls, dans lequel le capteur de fréquence du pouls est un capteur différent du capteur de pression, le procédé consistant à:
obtenir (131) des informations sur la fréquence du pouls du sujet à partir du capteur de fréquence du pouls;
adapter (133) un filtre de signal de pression en fonction des informations obtenues sur la fréquence du pouls du sujet, dans lequel le filtre de signal de pression est un filtre à moyenne mobile avec une largeur de fenêtre de moyenne réglée pour correspondre à la période cardiaque obtenue à partir des informations sur la fréquence du pouls du sujet;
commencer le gonflage du brassard;
obtenir (137) un signal de pression du capteur de pression, le signal de pression représentant la pression dans le brassard lorsque le brassard est gonflé;
filtrer (139) le signal de pression en utilisant le filtre de signal de pression adapté lorsque le signal de pression est obtenu, le filtrage comprenant les étapes consistant à:
faire passer le filtre à moyenne mobile sur le signal de pression, et soustraire le signal de pression résultant du signal de pression d'origine pour obtenir le signal de pression filtré; et traiter (141), à mesure que le signal de pression filtré est obtenu, le signal de pression filtré pour obtenir une mesure de pression sanguine pour le sujet.

2. Procédé selon la revendication 1, dans lequel l'étape d'obtention (131) d'informations sur la fréquence du pouls du sujet à partir du capteur de fréquence du pouls est effectuée avant de commencer le gonflage du brassard, et dans lequel, de préférence, l'étape d'adaptation (133) du filtre de signal de pression est également effectuée avant de commencer le gonflage du brassard.

3. Procédé selon la revendication 2, dans lequel les étapes d'obtention (131) d'informations sur la fréquence du pouls du sujet à partir du capteur de fréquence du pouls et d'adaptation (133) du filtre de signal de pression sont également effectuées pendant le gonflage du brassard.

4. Procédé selon l'une quelconque des revendications 1-3, le procédé comprenant en outre l'étape consistant à dégonfler (145) le brassard une fois que la mesure de pression sanguine a été obtenue.

5. Procédé selon l'une quelconque des revendications 1-4, le procédé comprenant en outre l'étape consistant à effectuer (145) une mesure de la pression sanguine pendant le dégonflage du brassard si l'étape de traitement (141) du signal de pression filtré pour obtenir une mesure de pression sanguine pour le sujet ne fournit pas une mesure de la pression sanguine ou une mesure acceptable.

6. Procédé selon l'une quelconque des revendications 1-5, dans lequel l'étape d'adaptation (133) du filtre de signal de pression comprend l'adaptation des caractéristiques de fréquence du filtre selon les informations obtenues sur la fréquence du pouls du sujet.

7. Produit de programme informatique comprenant un support lisible par ordinateur comportant du code lisible par ordinateur, le code lisible par ordinateur étant configuré de sorte que, lors de l'exécution par un ordinateur, processeur ou unité de commande adapté qui est destiné à être utilisé avec un moniteur de pression sanguine non invasif, NIBP, comprenant un brassard qui doit être placé autour d'un membre du sujet, une pompe pour gonfler le brassard et un capteur de pression pour mesurer la pression dans le brassard, dans lequel le moniteur NIBP est utilisé avec un capteur de fréquence du pouls, dans lequel le capteur de fréquence du pouls est un capteur différent du capteur de pression, l'ordinateur, le processeur ou l'unité de commande est amené à effectuer le procédé selon l'une quelconque des revendications 1-6.

8. Moniteur de pression sanguine non invasif, NIBP (50) pour mesurer la pression sanguine d'un sujet pendant le gonflage d'un brassard (58) lorsque le moniteur NIBP (50) est utilisé avec un capteur de fréquence du pouls (64), le moniteur NIBP comprenant un brassard (58) qui doit être placé autour d'un membre du sujet, une pompe (52) pour gonfler le brassard (58) et un capteur de pression (54) pour mesurer la pression dans le brassard (58), dans lequel le capteur de fréquence du pouls (64) est un capteur différent du capteur de pression (54), le moniteur NIBP (50) comprenant en outre:
une unité de commande (62) qui est configurée pour:
obtenir des informations sur la fréquence du pouls du sujet à partir du capteur de fréquence du pouls (64);
adapter un filtre de signal de pression en fonction des informations obtenues sur la fréquence du pouls du sujet, dans lequel le filtre de signal de pression est un filtre à moyenne mobile avec une largeur de fenêtre de moyenne réglée pour correspondre à la période cardiaque obtenue à partir des informations sur la fréquence du pouls du sujet;
commander la pompe (52) pour commencer à gonfler le brassard (58);
obtenir un signal de pression du capteur de pression (54), le signal de pression représentant la pression dans le brassard (58) lorsque le brassard (58) est gonflé;
filtrer le signal de pression en utilisant le filtre de signal de pression adapté lorsque le signal de pression est obtenu, le filtrage comprenant les étapes consistant à:
faire passer le filtre à moyenne mobile sur le signal de pression, et soustraire le signal de pression résultant du signal de pression d'origine pour obtenir le signal de pression filtré; et traiter le signal de pression filtré à mesure que le signal de pression filtré est obtenu pour obtenir une mesure de pression sanguine pour le sujet.

9. Moniteur NIBP (50) selon la revendication 8, dans lequel le capteur de fréquence du pouls (64) pour mesurer la fréquence du pouls du sujet et pour fournir des informations sur la fréquence du pouls à l'unité de commande (62) est compris dans le moniteur NIBP (50).

10. Moniteur NIBP (50) selon l'une quelconque des revendications 8-9, dans lequel l'unité de commande (62) est configurée pour obtenir les informations sur la fréquence du pouls du sujet et, de préférence, également pour adapter le filtre de signal de pression, avant de commander la pompe (52) pour commencer à gonfler le brassard (58).

11. Moniteur NIBP (50) selon l'une quelconque des revendications 8-10, dans lequel l'unité de commande (62) est en outre configurée pour obtenir des informations sur la fréquence du pouls du sujet à partir du capteur de fréquence du pouls (64) et pour adapter le filtre de signal de pression lors du gonflage du brassard (58) .

12. Moniteur NIBP (50) selon l'une quelconque des revendications 8-11, dans lequel l'unité de commande (62) est configurée pour adapter le filtre de signal de pression en adaptant les caractéristiques de fréquence du filtre selon les informations obtenues sur la fréquence du pouls du sujet.
